(19) Europäisches Patentamt
European Patent Office
Office européen des brevets

(11) **EP 1 772 103 B1**

(12) **EUROPEAN PATENT SPECIFICATION**

(45) Date of publication and mention
of the grant of the patent:
**13.05.2009 Bulletin 2009/20**

(51) Int Cl.:
*A61B 8/08* (2006.01)

(21) Application number: **06021015.0**

(22) Date of filing: **06.10.2006**

(54) **Ultrasound imaging system for extracting volume of an object from an ultrasound image and method for the same**

Ultraschallabbildungssystem zur Gewinnung vom Volumen eines Objektes aus einem Ultraschallbild und Verfahren dafür

Système d'imagerie ultrasonore pour l'extraction du volume d'un objet à partir d'une image ultrasonore et procédé correspondant

(84) Designated Contracting States:
**DE FR IT**

(30) Priority: **07.10.2005 KR 20050094318**

(43) Date of publication of application:
**11.04.2007 Bulletin 2007/15**

(73) Proprietor: **MEDISON CO., LTD.**
**Kangwon-do 250-870 (KR)**

(72) Inventors:
• **Kim, Nam Chul**
  **Suseong-gu, Daegu 706-767 (KR)**
• **Oh, Jong Hwan**
  **Gyeongsangbuk-do 730-916 (KR)**
• **Kim, Sang Hyun**
  **Busan 612-778 (KR)**
• **Kwak, Jong In**
  **Daegu 703-811 (KR)**
• **Ahn, Chi Young**
  **Discusser&Medison Bldg.**
  **Seoul 135-280 (KR)**

(74) Representative: **Schmid, Wolfgang**
  **Lorenz & Kollegen**
  **Patent- und Rechtsanwaltskanzlei**
  **Alte Ulmer Strasse 2**
  **D-89522 Heidenheim (DE)**

(56) References cited:
**EP-A- 1 582 150**

• **CHEN C-H ET AL: "3D RECONSTRUCTION OF THE PROSTATE FROM TRANSRECTAL ULTRASOUND IMAGES" BIOMEDICAL ENGINEERING - APPLICATIONS, BASIS & COMMUNICATIONS, vol. 11, no. 1, 25 February 1999 (1999-02-25), pages 1-10, XP001040042 ISSN: 1016-2356**
• **SAKAS G ET AL ASSOCIATION FOR COMPUTING MACHINERY: "EXTRACTING SURFACES FROM FUZZY 3D-ULTRASOUND DATA" COMPUTER GRAPHICS PROCEEDINGS. LOS ANGELES, AUG. 6 - 11, 1995, COMPUTER GRAPHICS PROCEEDINGS (SIGGRAPH), NEW YORK, IEEE, US, 6 August 1995 (1995-08-06), pages 465-474, XP000546259 ISBN: 0-89791-701-4**
• **CENA, SPADACCINI: "A wavelet based data visualisation system" PROC OF THE EIGHTH COMPUTER SCIENCE RESEARCH CONFERENCE, April 1997 (1997-04), pages 37-41, XP002414845**
• **XIANG S H ET AL: "Three dimensional ultrasound image reconstruction using nonlinear wavelet thresholding" ENGINEERING IN MEDICINE AND BIOLOGY SOCIETY, 1996. BRIDGING DISCIPLINES FOR BIOMEDICINE., 18TH ANNUAL INTERNATIONAL CONFERENCE OF THE IEEE AMSTERDAM, NETHERLANDS 31 OCT.-3 NOV. 1996, NEW YORK, NY, USA,IEEE, US, vol. 5, 31 October 1996 (1996-10-31), pages 2238-2239, XP010261659 ISBN: 0-7803-3811-1**

- BOUKERROUI D ET AL: "Segmentation of ultrasound images--multiresolution 2D and 3D algorithm based on global and local statistics" PATTERN RECOGNITION LETTERS, NORTH-HOLLAND PUBL. AMSTERDAM, NL, vol. 24, no. 4-5, February 2003 (2003-02), pages 779-790, XP004391216 ISSN: 0167-8655
- WEN-LI LEE ET AL: "Ultrasonic Liver Tissues Classification by Fractal Feature Vector Based on M-Band Wavelet Transform" IEEE TRANSACTIONS ON MEDICAL IMAGING, IEEE SERVICE CENTER, PISCATAWAY, NJ, US, vol. 22, no. 3, March 2003 (2003-03), pages 382-392, XP011076436 ISSN: 0278-0062
- DINGGANG SHEN ET AL: "Segmentation of Prostate Boundaries From Ultrasound Images Using Statistical Shape Model" IEEE TRANSACTIONS ON MEDICAL IMAGING, IEEE SERVICE CENTER, PISCATAWAY, NJ, US, vol. 22, no. 4, April 2003 (2003-04), pages 539-551, XP011076455 ISSN: 0278-0062

**Description**

FIELD OF THE INVENTION

The present invention generally relates to an ultrasound imaging system and a method for processing an ultrasound image, and more particularly to an ultrasound imaging system for automatically extracting volume data of a prostate and a method for the same.

BACKGROUND OF THE INVENTION

An ultrasound diagnostic system has become an important and popular diagnostic tool due to its wide range of applications. Specifically, due to its non-invasive and non-destructive nature, the ultrasound diagnostic system has been extensively used in the medical profession. Modem high-performance ultrasound diagnostic systems and techniques are commonly used to produce two or three-dimensional (2D or 3D) diagnostic images of a target object. The ultrasound diagnostic system generally uses a wide bandwidth transducer to transmit and receive ultrasound signals. The ultrasound diagnostic system forms ultrasound images of the internal structures of the target object by electrically exciting the transducer to generate ultrasound pulses that travel into the target object. The ultrasound pulses produce ultrasound echoes since they are reflected from a discontinuous surface of acoustic impedance of the internal structure, which appears as discontinuities to the propagating ultrasound pulses. The various ultrasound echoes return to the transducer and are converted into electrical signals, which are amplified and processed to produce ultrasound data for an image of the internal structure.

A prostate is a chestnut-sized exocrine gland in a male and located just below a bladder. An ejaculation duct and a urethra pass through a center of the prostate. Thus, if the prostate is inflamed or enlarged, it can cause various urinary problems. Prostate-related diseases can often occur in men over 60 years old. In the U.S., prostate cancer is the second leading cause of cancer death in men. It is predicted that the number of prostate patients will increase in the future since more men are becoming older these days. However, if discovered early, the prostate cancer can be treated. Thus, the early diagnosis is very important.

The ultrasound diagnostic system is widely employed for the early diagnosis and treatment of the prostate cancer due to its lower cost, portability, real-time imaging and the like. Mostly, a contour of the prostate is manually extracted from cross-sectional images of the prostate displayed on the screen of the ultrasound diagnostic system to thereby obtain volume information of the prostate. In such a case, it takes a long time to extract the prostate contour. Further, different extraction results are obtained when one user repeatedly extracts contours from the same sectional image or when different users extract contours from the same sectional image.

Recently, methods for automatically or semi-automatically extracting the prostate contour from the ultrasound images have been studied extensively.

Hereinafter, a conventional method for extracting the prostate contour from the ultrasound sectional images using wavelet transform and snakes algorithm will be described.

First, images are obtained at respective scales by a wavelet transform, wherein an image is repeatedly filtered with a low-pass filter and a high-pass filter horizontally and vertically. Among those images, an image obtained at a specific scale, in which prostate contour is distinguished from speckle noises, is employed to manually draw a first draft contour of the prostate thereon.

Next, on an image obtained at the scale (one step lower than the specific scale), the more accurate contour of the prostate is detected by using the snakes algorithm based on the first draft contour. By repeating this down to the lowest scale, the prostate contour can be more accurately detected step by step.

The above-mentioned conventional method has merits in that speckle noises can be reduced in the low-pass image obtained by the wavelet transform and the accuracy of the contour can be assured by using relationship between wavelet coefficients in different bands. On the other hand, the conventional method is disadvantageous in that the user has to manually draw draft contours on all the 2D cross-sectional images obtained from the 3D volume in the snakes algorithm and the contour detection results considerably depend on snakes variables.

On the other hand, there has been proposed another method for extracting the prostate contour from the ultrasound cross-sectional images by manually connecting edges extracted therefrom.

In such a method, the ultrasound cross-sectional images are first filtered with a stick-shaped filter and an anisotropic diffusion filter to reduce speckle noises.

Next, edges are automatically extracted from the images based on pre-input information such as the shape of the prostate and echo patterns. Then, the user manually draws the prostate contour based on the extracted edges. According to such a method, substantially accurate and consistent results can be obtained regardless of the users. However, the time required for extracting the prostate contour can still be long depending on the sizes of input images and the stick-shaped filter. Thus, the user has to intervene in drawing the prostate contour on all ultrasound cross-sectional images.

Chen et al. (3D reconstruction of the prostate from transrectal ultrasound images, Biomed Eng-App Bas C, 11 (1), 1999) describe an ultrasound imaging system for forming 3D volume data of a target object, comprising: a three-dimensional (3D) image providing unit for providing a 3D ultrasound image; a preprocessing unit adapted to form a number of two-dimensional images, an edge extraction unit, a control point determining unit adapted to determine control points and a rendering unit adapted to form 3D volume data of the target object.

SUMMARY OF THE INVENTION

The present invention provides an ultrasound imaging system for automatically extracting volume data of a prostate with wavelet transformation and a support vector machine and a method for the same.

In accordance with one aspect of the present invention, there is provided an ultrasound imaging system for forming 3D volume data of a target object, which includes: a three-dimensional (3D) image providing unit for providing a 3D ultrasound image; a pre-processing unit for forming a number of two-dimensional (2D) images from the 3D ultrasound image and normalizing the 2D images, respectively, to form normalized 2D images; an edge extraction unit for forming wavelet-transformed images of the normalized 2D images at a number of scales, forming edge images by averaging the wavelet-transformed images at a number of scales, and thresholding the edge images; a control point determining unit for determining control points by using a support vector machine (SVM) based on the normalized 2D images, the wavelet-transformed images and the thresholded edge images; and a rendering unit for forming 3D volume data of the target object by 3D rendering based on the control points.

In accordance with another aspect of the present invention, there is provided a method for extracting 3D volume data of a target object, which includes: forming a number of two-dimensional (2D) images from a three-dimensional (3D) image; normalizing the 2D images to create normalized 2D images, respectively; forming wavelet-transformed images of the normalized 2D images at a number of scales; forming edge images by averaging the wavelet-transformed images at a number of scales; thresholding the edge images; determining control points by using a support vector machine (SVM) based on the normalized 2D images, the wavelet-transformed images and the thresholded edge images; and forming 3D volume data of the target object by 3D rendering based on the control points.

In accordance with the present invention, there are provided an ultrasound imaging system and method for extracting volume data of an object from 3D ultrasound image data by using wavelet transform and SVM. Thus, it is possible to obtain a clear contour of the object while reducing noises in the edge image formed by averaging wavelet-transformed images at respective scales.

BRIEF DESCRIPTION OF THE DRAWINGS

The above and other objects and features of the present invention will become apparent from the following description of an embodiment given in conjunction with the accompanying drawings, in which:

FIG. 1 is a block diagram showing an ultrasound imaging system constructed in accordance with one embodiment of the present invention;

FIG. 2 is a diagram for explaining acquisition of 2D cross-sectional images from 3D image;

FIGS. 3A and 3B are ultrasound pictures showing 2D cross-sectional images obtained from different volumes;

FIGS. 4A and 4B are ultrasound pictures obtained by normalizing the pixel values of the 2D cross-sectional images shown in FIGS. 3A and 3B;

FIG 5 is an exemplary diagram showing a wavelet transform process;

FIG. 6A is an ultrasound picture showing a pre-processed prostate image;

FIGS. 6B to 6D are ultrasound pictures showing images at scales $2^2$, $2^3$ and $2^4$ obtained by performing wavelet transform on the pre-processed prostate image;

FIG. 7A shows a wavelet-transformed image at scale $2^3$;

FIG. 7B shows a waveform in the 115th horizontal line in the wavelet-transformed image shown in FIG 7A;

FIG. 8A shows an edge image obtained by averaging wavelet-transformed images at scales $2^2$, $2^3$ and $2^4$;

FIG. 8B shows a waveform in the 115th horizontal line in the edge image shown in FIG. 8A;

FIG. 9 is an ultrasound picture showing a thresholded edge image;

FIG. 10 shows radial lines arranged around a center of the prostate;

FIGS. 11A to 11F show images obtained by a method for extracting a 3D ultrasound prostate volume in accordance with one embodiment of the present invention; and

FIG. 12 is a graph showing average absolute distances for cross-sectional images.

## DETAILED DESCRIPTION OF THE PRESENT INVENTION

Hereinafter, an ultrasound imaging system and method for automatically extracting a volume of a target object (for example, a prostate) in accordance with the present invention will be described with reference to the accompanying drawings.

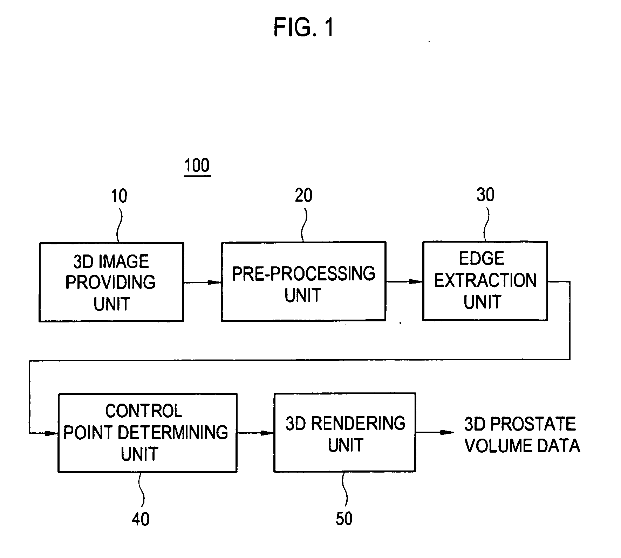

Referring now to FIG. 1, an ultrasound imaging system 100 for forming volume data of a target object in accordance with one embodiment of the present invention includes a 3D ultrasound image providing unit 10, a pre-processing unit 20, an edge extraction unit 30, a control point determining unit 40 and a 3D rendering unit 50. The 3D image providing unit 10 can be a memory or a probe. The pre-processing unit 20, the edge extraction unit 30, the control point determining unit 40 and the 3D rendering unit 50 can be embodied with one processor. The control point determining unit 40 includes a support vector machine (SVM).

Here, an edge is a point where the discontinuity of brightness appears. Further, a boundary is a contour of the target object, for example, a prostate.

1. Pre-processing

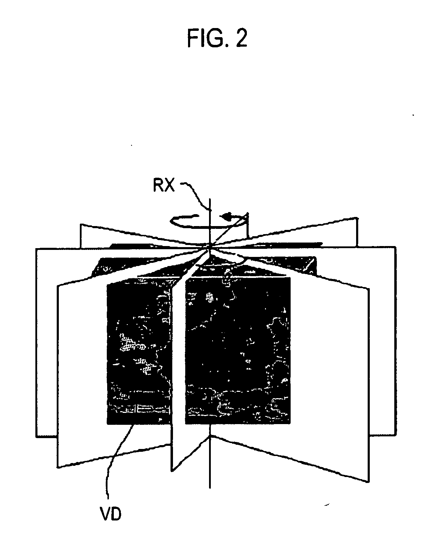

A method for acquiring 2D cross-sectional images from the 3D image will be described with reference to FIG 2. In FIG 2, a rotation axis RX is a virtual axis passing through a center of the 3D image VD provided with unit 10. The pre-processing unit 20 produces a number of the 2D cross-sectional images by rotating the 3D image by specified angles θ around the rotation axis RX. In this embodiment, six 2D cross-sectional images with an image size of 200 x 200 are produced at every 30 degrees around the rotation axis RX.

Next, the average and standard deviation of a pixel value (preferably, brightness and contrast) of each 2D cross-sectional image are normalized. At this time, a background having brightness of zero in the 2D ultrasound image is excluded from normalization. In this embodiment, the average and standard deviation are set to be 70 and 40, respectively.

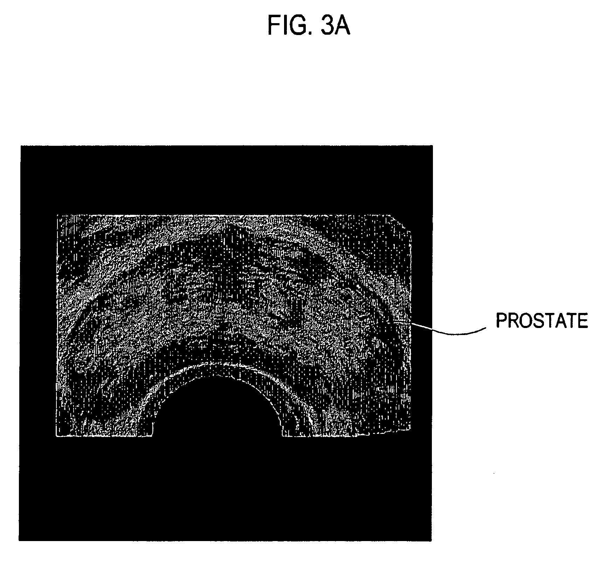

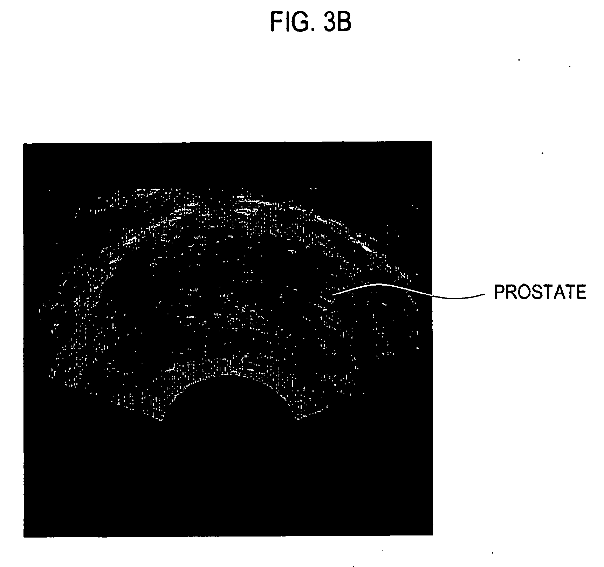

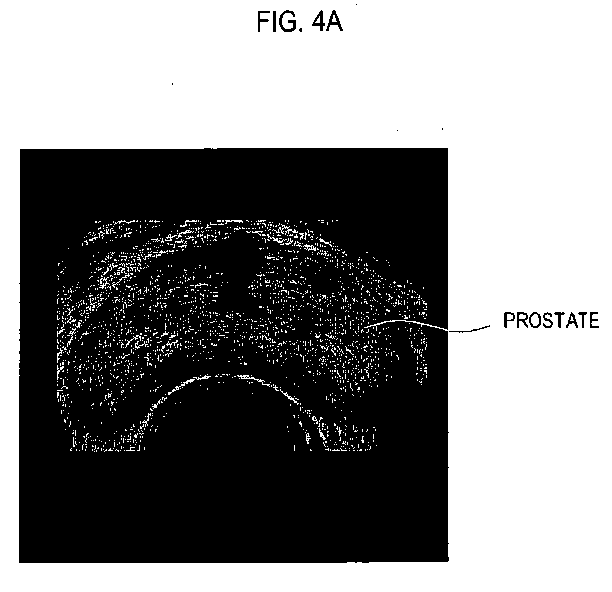

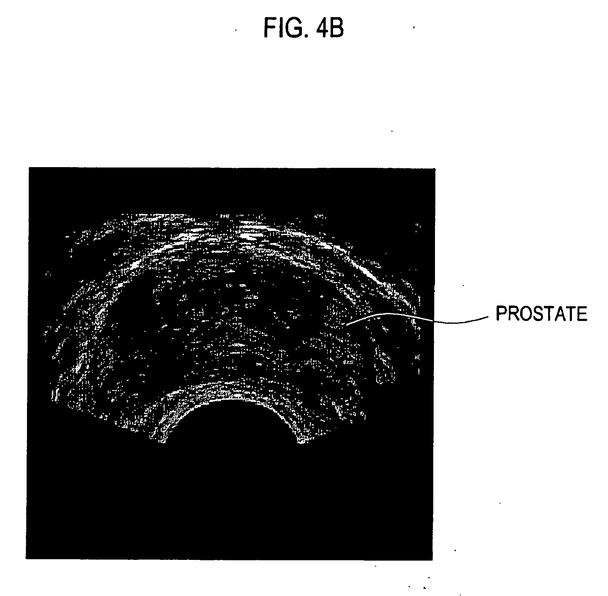

FIGS. 3A and 3B are ultrasound pictures showing 2D cross-sectional images obtained from different 3D images. FIGS. 4A and 4B are ultrasound pictures obtained by normalizing the 2D cross-sectional images shown in FIGS. 3A and 3B. As shown in FIGS. 4A and 4B, normalization yields images having uniform brightness characteristics regardless of 3D input images.

2. Edge extraction

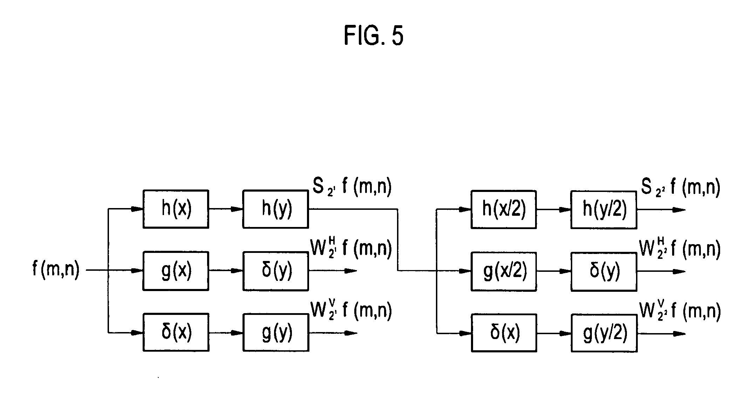

The edge extraction unit 30 decomposes the 2D cross-sectional image into a set of sub-band images. Namely, the edge extraction unit 30 applies the wavelet decomposition to the normalized 2D cross-sectional images provided from the pre-processing unit 20 in the manner shown in FIG 5 by using Equations 1 to 3.

$$W_{2^j}^{H} f(m,n) = S_{2^{j-1}} f(m,n) * g(m/2^{j-1}) * \delta(n) \qquad \text{Eq. 1}$$

$$W_{2^j}^{V} f(m,n) = S_{2^{j-1}} f(m,n) * \delta(m) * g(n/2^{j-1}) \qquad \text{Eq. 2}$$

$$S_{2^j} f(m,n) = S_{2^{j-1}} f(m,n) * h(m/2^{j-1}) * h(n/2^{j-1}) \qquad \text{Eq. 3}$$

In Equations 1 to 3, $f(m,n)$ represents a pre-processed image; h(n) and g(n) respectively represent a low-pass filter and a high-pass filter for wavelet transform; and δ(x) represents an impulse function. The superscripts $H$ and $V$ denote horizontal and vertical filtering, respectively. Further, $W_{2^j}^{H} f(m,n)$ and $W_{2^j}^{V} f(m,n)$ respectively represent high-pass images containing vertical and horizontal edge information at scale $2^j$, whereas $S_{2^j}f(m,n)$ represents a low-pass image at scale $2^j$ obtained from the pre-processed image $f(m,n)$. The pre-processed image $f(m,n)$ can be represented as $S_{2^0}f(m,n)$

Next, the results of the wavelet transform at scale $2^j$ are applied to Equation 4 to obtain images $M_{2^j}f(m,n)$ at scale $2^j$.

$$M_{2^j}f(x,y) = \sqrt{|W_{2^j}^H f(m,n)|^2 + |W_{2^j}^V f(m,n)|^2} \qquad \text{Eq. 4}$$

$$Mf(m,n) = \frac{1}{3}\sum_{j=2}^{4}\frac{M_{2^j}f(m-d_{2^j},n-d_{2^j})}{\max_{(m,n)}\left(M_{2^j}f(m-d_{2^j},n-d_{2^j})\right)} \qquad \text{Eq. 5}$$

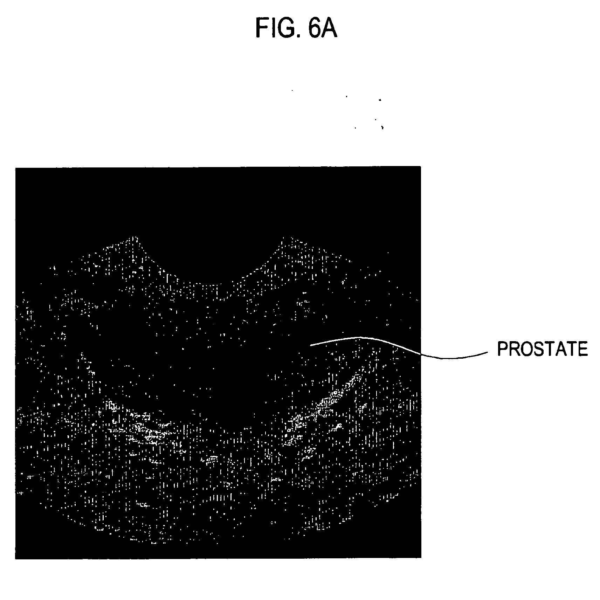

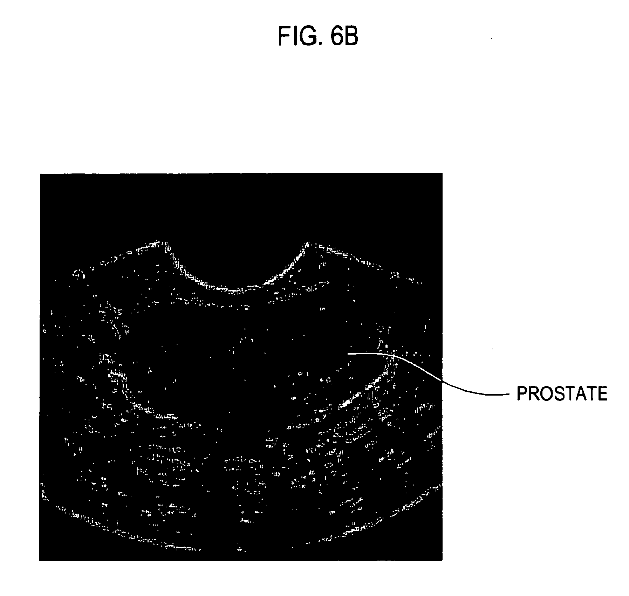

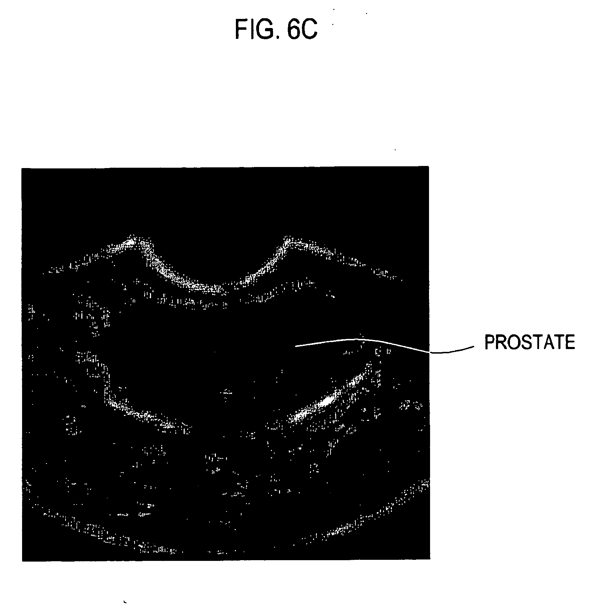

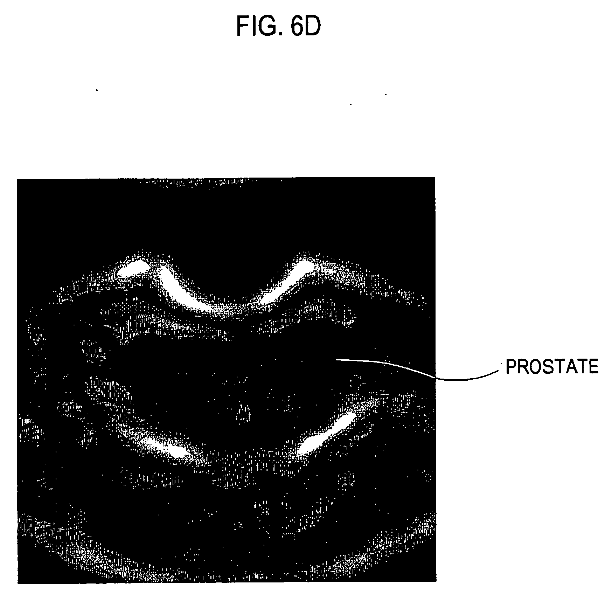

FIG. 6A shows a pre-processed prostate image and FIGS. 6B to 6D respectively show wavelet-transformed images $M_{2^2}f(m,n)$, $M_{2^3}f(m,n)$ and $M_{2^4}f(m,n)$ at scales $2^2$, $2^3$ and $2^4$ obtained by performing wavelet transform on the pre-processed prostate image. As shown in FIGS. 6B to 6D, the prostate and noises can be apparently discriminated as the scale is increased. However, the boundary of the prostate becomes too unclear to accurately capture a position of the boundary.

Then, in order to reduce noises in the image and clear the boundary of the prostate, wavelet-transformed images at each scale are averaged by using Equation 5.

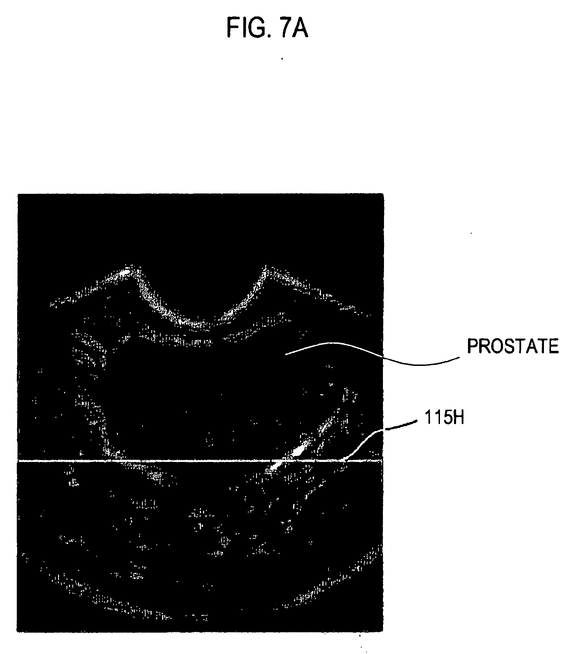

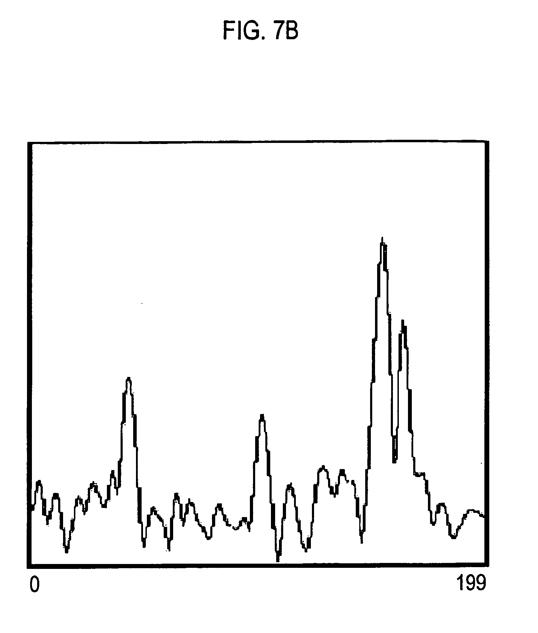

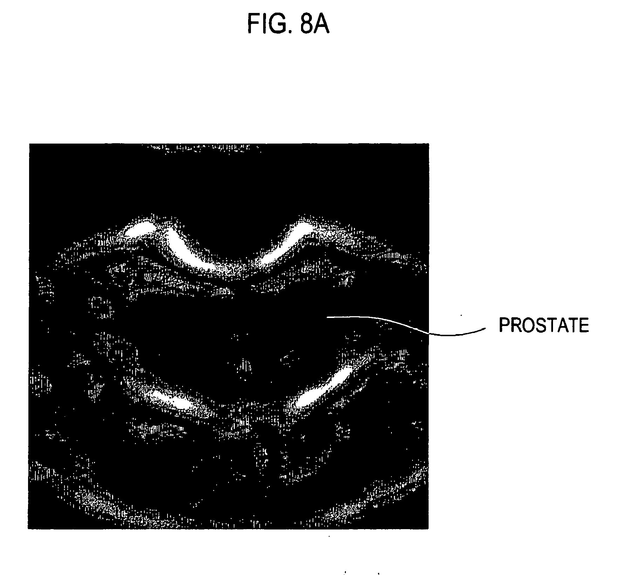

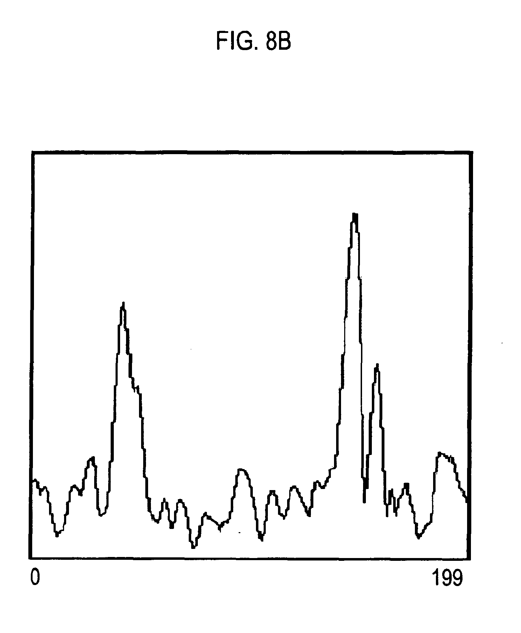

In Equation 5, $\max_{(m,n)}(\cdot)$ is an operator for computing a maximum pixel value in images, and $Mf(m,n)$ represents an edge image obtained by averaging the wavelet-transformed images. On the other hand, since the centers of the filters are delayed by 1/2 in Equations 1 to 3, the images at the respective scales are horizontally and vertically compensated by $d_{2^j} = (1/2)\cdot\sum_{n=1}^{j}2^{n-1}$ before averaging, so that the boundary positions of the prostate can be set to be equal regardless of scales. FIGS. 7A, 7B, 8A and 8B show effects obtained when wavelet-transformed images are averaged. FIG. 7A shows the wavelet-transformed image at scale $2^3$, whereas FIG. 7B shows a waveform of the 115th horizontal line in the image shown in FIG. 7A. FIG. 8A shows an edge image obtained by averaging wavelet-transformed images at scales $2^2$, $2^3$ and $2^4$, whereas FIG. 8B shows a waveform of the 115th horizontal line in the edge image shown in FIG 8A. By comparing FIGS. 7A and 8A, it can be seen that the edge image (FIG. 8A) obtained by averaging shows less noises and a clearer boundary than the image (FIG. 7A), which has only undergone the wavelet transformation.

Next, in order to reduce noises in the edge image obtained by averaging, the brightness of the edge image is thresholded with Equation 6.

$$M_T f(m,n) = \begin{cases} Mf(m,n) & \text{if } \quad Mf(m,n) > Th \\ 0 & otherwise \end{cases} \qquad \text{Eq. 6}$$

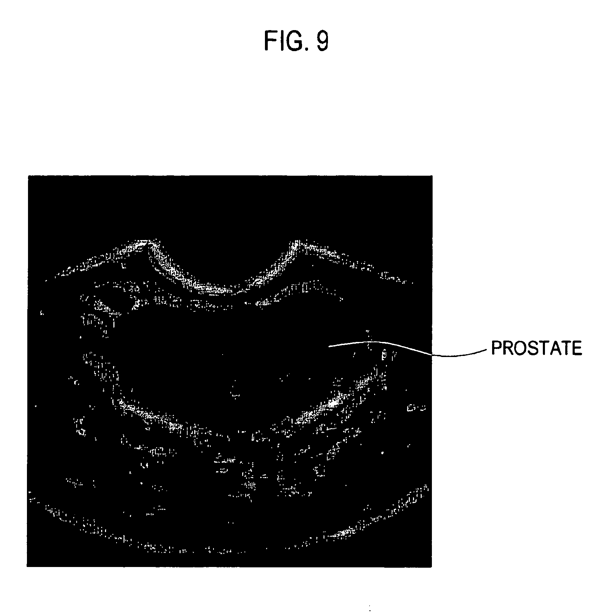

In Equation 6, Th represents the threshold, and $M_T f(m,n)$ represents a thresholded edge image obtained from the edge image $Mf(m,n)$. FIG. 9 shows the thresholded edge image.

In short, an edge image with less speckle noises can be obtained by the above-mentioned edge extraction, wherein images at respective scales obtained by performing wavelet transform on a 2D cross-sectional image are averaged to form an edge image and the edge image is then thresholded.

3. Determining of control points

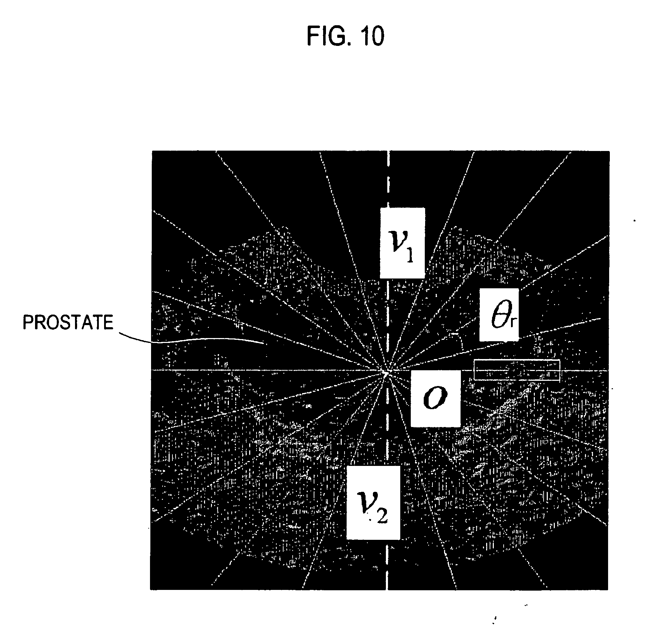

Control points are determined based on the fact that an inner portion of the prostate is darker than an external portion thereof in the ultrasound image. The control points will be used to obtain the prostate volume through 3D rendering. The control point determining unit 40 is provided with the thresholded edge image $M_T f(m,n)$ and the wavelet-transformed images from the edge extraction unit 30. Such a unit then determines a number of control points at which the prostate contour intersects predetermined directional lines, preferably, radial lines in the pre-processed image $f(m,n)$. FIG. 10 shows the radial lines arranged around a center point $O$ of the prostate, from which the radial lines are originated. The center point $O$ is determined with a mid-point between two reference points $v_1$ and $v_2$ selected by the user. Hereinafter, a method of determining the control points will be described in detail.

The control point determining unit 40 searches first candidate points having brightness greater than zero along the radial lines, respectively with the thresholded edge image $M_T f(m,n)$.

Next, internal and external windows having a size of *M* x *N* are set around each of the first candidate points in a low-pass sub-band image at a predetermined scale, produced by the wavelet transform. The internal and external windows are adjacent each other over the first candidate point. Then, by comparing averages of the brightness of the internal and external windows, the first candidate points of which the external window has greater average brightness than the internal window are set as second candidate points. In this embodiment, the second candidate points are selected by using the wavelet-transformed low-pass sub-band image $S_{23}f(m,n)$ at scale $2^3$.

Next, feature vectors at the second candidate points are generated by using a support vector machine (SVM) in order to classify the second candidate points into two groups of points, wherein one group of points have characteristics of the control points and the other group of points do not. For this, first, internal and external windows having a size of *M* x *N* are set around each of the second candidate points along a radial direction on the pre-processed image $f(m,n)$. Then, averages and standard deviations of the windows in the pre-processed image, block difference invert probabilities (BDIP), averages of block variation of local correlation coefficients (BVLC) are obtained to generate the feature vectors expressed by the following Equation 7.

$$\mathbf{h} = [\mu_{out}(f), \mu_{in}(f), \sigma_{out}(f), \sigma_{in}(f), \mu_{out}(D), \mu_{in}(D), \mu_{out}(V), \mu_{in}(V)] \qquad \text{Eq. 7}$$

In Equation 7, $\mu_{out(in)}(\cdot)$ and $\sigma_{out(in)}(\cdot)$ respectively represent the average and standard deviation in the external (internal) window, and *D* and *V* denote BDIP and BVLC, respectively, for the pre-processed image.

BDIP is defined as a ratio of a sum of values, which are obtained by subtracting the pixel values in the block from the maximum pixel value in the block, to the maximum pixel value in the block. The BVLC is defined with a difference between the maximum and the minimum correlation coefficients among four local correlation coefficients at one pixel in a block. The BDIP and BVLC are well-known and, thus, detailed description thereof will be omitted herein.

After obtaining the feature vectors of all the second candidate points as described above, in order to prevent specified components of the feature vectors from affecting SVM classification, respective components of the feature vectors are normalized as Equation 8.

$$\mathbf{x} = \mathbf{h} / \sigma \qquad \text{Eq. 8}$$

In Equation 8, " / " denotes component-wise division of two vectors; σ is a vector defined with a standard deviation calculated from the columns of the respective components of h; and x is a normalized feature vector.

Next, the most appropriate point for the control points among the second candidate points is determined in each radial direction by using a trained SVM based on the brightness in the thresholded edge image $M_T f(m,n)$. Then, the detected points are determined to be third candidate points in respective radial directions. If all the second candidate points in a specified radial direction are determined not to be appropriate for the control points, a brightest point among the second candidate points of the radial direction in the edge image is selected as the third candidate point.

On the other hand, data used for training the SVM contain points divided into two groups, wherein one group including the points, artificially determined by the user and has characteristics of the control points, and the other group including the points that do not meet the characteristics of the control point. The feature vectors are extracted from the points of the two groups by using Equation 8 to train the SVM. In this embodiment, to train the SVM, 60 points with characteristics of the control points and 60 points with no characteristics of the control points are extracted from images unrelated to the prostate. Further, windows set in the images for extracting the feature vectors have a size of 9 x 3.

Then, while taking a basic contour of the target object into account, that is, supposing that the contour of the prostate curves gently, the positions of the third candidate points are readjusted as expressed by Equation 9.

$$\hat{P}_i = \frac{P_{i-1} + P_{i+1}}{2}, \quad \text{if } |P_i - P_{i-1}| > \frac{1}{N} \sum_{i=1}^{N} |P_i - P_{i-1}| \qquad \text{Eq. 9}$$

In Equation 9, $P_i$ represents the positions of the third candidate points in the $i^{th}$ radial line.

Then, points corresponding to the edges of the greatest brightness within specified ranges, which include the readjusted third candidate points, are finally determined as the control points in the respective radial directions.

The 3D rendering unit 50 constructs a 3D wire frame of a polyhedron object (i.e., the prostate) based on the determined control points to obtain an ultrasound prostate volume by using surface-based rendering techniques.

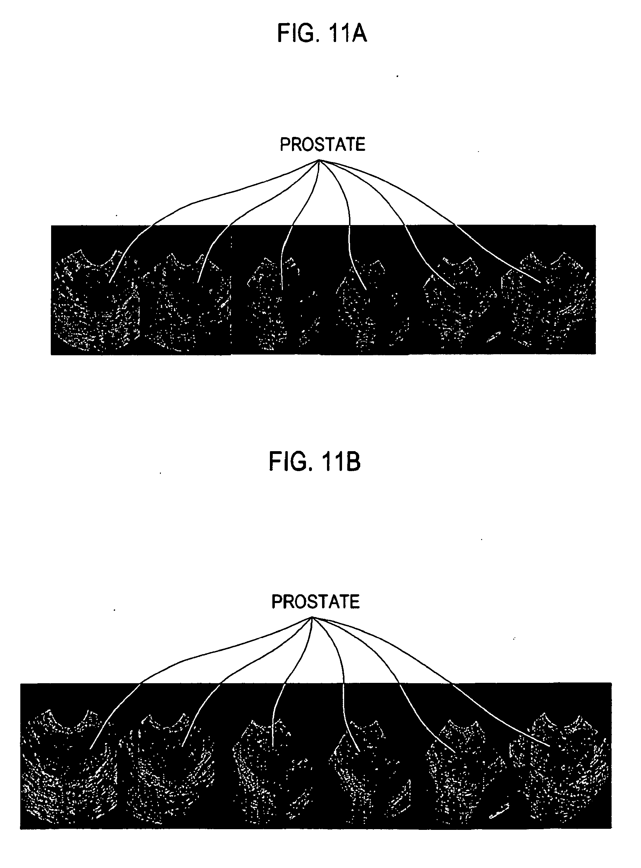

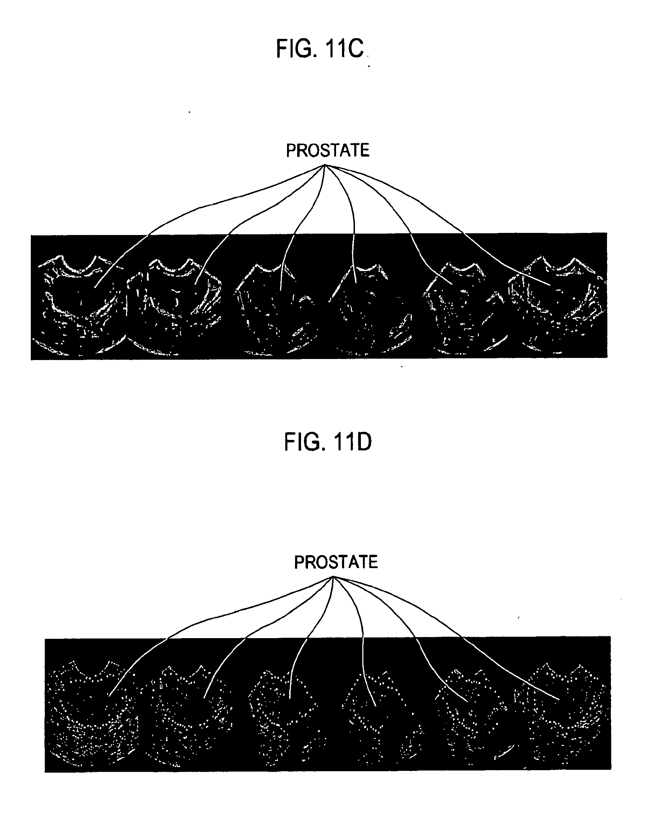

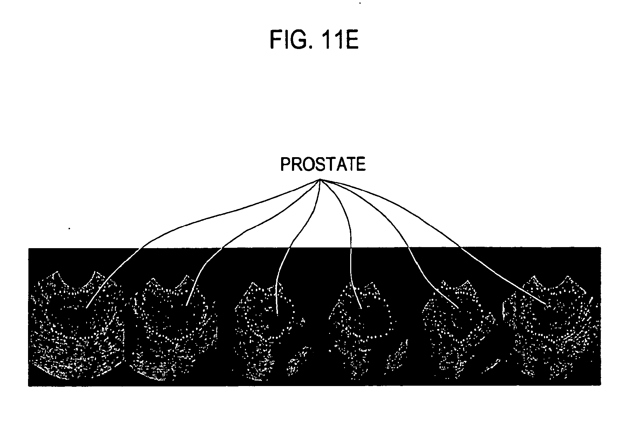

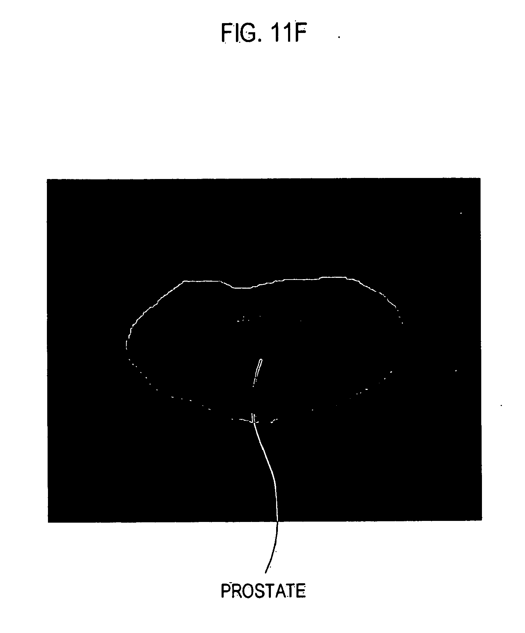

FIGS. 11A to 11F show images obtained at each step in a method for extracting a 3D ultrasound prostate volume in accordance with the present invention. FIG. 11A shows the 2D cross-sectional images obtained at every 30 degrees by rotating the 3D image. FIG. 11B shows the images obtained by normalizing the brightness of the images shown in FIG. 11A. By comparing FIGS. 11A and 11B, it can be seen that the brightness normalized images (FIG. 11B) show clearer boundaries than FIG. 11A. FIG. 11C shows images configured with the thresholded edge image $M_T f(m, n)$, which was obtained by averaging and thresholding images $\{M_{2j} f(m,n)\}$ ($2 \leq j \leq 4$). FIG. 11D shows the third candidate points determined by the SVM. FIG. 11E shows images containing the readjusted third candidate points, which have gentle contours compared to those in FIG. 11D. Finally, FIG 11F shows the 3D prostate volume extracted from the ultrasound image based on the control points by using the surface-based rendering techniques.

The performance of the 3D prostate volume extraction in accordance with the present invention can be evaluated by using an average absolute distance defined as Equation 10.

$$e_M = \frac{1}{N} \sum_{i=0}^{N-1} \min_j |b_j - a_i| \qquad \text{Eq. 10}$$

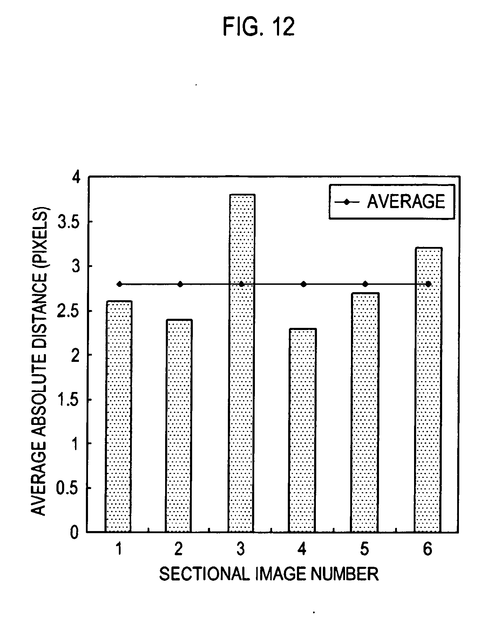

In Equation 10, $e_M$ represents average absolute distances; $a_i$ represents control points on the contour $A = \{a_0, a_1,..., a_{N-1}\}$ that is extracted manually, and $b_j$ represents control points on the contour $B=\{b_0,b_1,\cdots b_{N-1}\}$ that is obtained by using the above-mentioned method. FIG. 12 shows the average absolute distances $e_M$ for cross-sectional images. Referring to FIG 12, the average absolute distances $e_M$ range from about 2.3 to 3.8 pixels and average 2.8 pixels. It exhibits a similar performance as the conventional method of manually extracting the contour, in which $e_M$ is about 2 pixels on the average.

In the ultrasound imaging system and method of the present invention, the volume of the prostate is extracted from the 3D ultrasound image with the wavelet transformation and the SVM. The wavelet-transformed images at same scale are averaged to reduce noise and to obtain apparent boundary of the object on the edge image.

While the present invention has been described and illustrated with respect to an embodiment of the invention, it will be apparent to those skilled in the art that variations and modifications are possible without deviating from the broad principles and teachings of the present invention which should be limited solely by the scope of the claims appended hereto.

**Claims**

1. An ultrasound imaging system for forming 3D volume data of a target object, comprising:

   a three-dimensional (3D) image providing unit (10) for providing a 3D ultrasound image;
   a pre-processing unit (20) adapted to form a number of two-dimensional (2D) images from the 3D ultrasound image and normalize the 2D images to form normalized 2D images;
   an edge extraction unit (30) adapted to form wavelet-transformed images of the normalized 2D images at a number of scales, the edge extraction unit further being adapted to form edge images by averaging the wavelet-transformed images at a number of scales and threshold the edge images;
   a control point determining unit (40) adapted to determine control points by using a support vector machine (SVM) based on the normalized 2D images, the wavelet-transformed images and the thresholded edge images; and
   a rendering unit (50) adapted to form 3D volume data of the target object by 3D rendering based on the control points.

2. The ultrasound imaging system of Claim 1, wherein the target object is a prostate.

3. The ultrasound imaging system of Claim 1, wherein the pre-processing unit is adapted to normalize an average and a standard deviation of said 2D images to form the normalized 2d images.

4. The ultrasound imaging system of Claim 3, wherein the rendering (50) is adapted to render at least one of the normalized 2D images, the wavelet-transformed images and the thresholded edge images based on the control points.

5. The ultrasound imaging system of Claim 3, wherein the control point determining unit (40) is adapted to determine the control points by:

arranging a plurality of radial lines around a center of the target object in the thresholded edge images;
selecting first candidate points with a brightness greater than zero on each of the radial lines;
setting internal and external windows around each of the first candidate points;
comparing averages of the brightness in internal and external windows in the wavelet-transformed image at a predetermined scale;
selecting second candidate points with a greater brightness average in the external window than in the internal window among the first candidate points on each of the radial lines;
generating feature vectors of the second candidate points in the normalized 2D image and normalizing components of the feature vectors;
training the SVM by using the normalized feature vectors;
selecting third candidate points with the greatest brightness among the second candidate points on each of the radial lines in the thresholded edge images by using the trained SVM;
readjusting positions of the third candidate points based on a basic contour of the target object; and
determining an edge part of the target object with the greatest brightness within a predetermined distance among the readjusted third candidate points as the control points.

6. A method for extracting 3D volume data of a target object, comprising:

forming a number of two-dimensional (2D) images from a three-dimensional (3D) image;
normalizing the 2D images to create normalized 2D images;
forming wavelet-transformed images of the normalized 2D images at a number of scales;
forming edge images by averaging the wavelet-transformed images at a number of scales;
thresholding the edge images;
determining control points by using a support vector machine (SVM) based on the normalized 2D images, the wavelet-transformed images and the thresholded edge images; and
forming 3D volume data of the target object by 3D rendering based on the control points.

7. The method of Claim 6, wherein the target object is a prostate.

8. The method of Claim 6, wherein normalizing the 2D images includes normalizing an average and a deviation of brightness in the 2D images.

9. The method of Claim 8, wherein the 3D volume data is formed by rendering at least one of the normalized 2D images, the wavelet-transformed images and the thresholded edge images based on the control points.

10. The method of Claim 9, wherein determining the control points includes:

arranging a plurality of radial lines around a center of the target object in the thresholded edge images;
selecting first candidate points with a brightness greater than zero on each of the radial lines;
setting internal and external windows around each of the first candidate points;
comparing averages of the brightness in internal and external windows in the wavelet-transformed image at a predetermined scale;
selecting second candidate points with a greater brightness average in the external window than in the internal window among the first candidate points on each of the radial lines;
generating feature vectors of the second candidate points in the normalized 2D image and normalizing components of the feature vectors;
training the SVM by using the normalized feature vectors;
selecting third candidate points with the greatest brightness among the second candidate points on each of the radial lines in the thresholded edge image by using the trained SVM;
readjusting positions of the third candidate points based on a basic contour of the target object; and
determining an edge part of the target object with the greatest brightness within a predetermined distance among the readjusted third candidate points as the control points.

**Patentansprüche**

1. Ultraschallabbildungssystem zur Bildung von 3D-Volumendaten eines Zielobjekts, welches Folgendes aufweist:

   eine dreidimensionale (3D) Bildbereitstellungseinheit (10) zum Erzeugen eines 3D-Ultraschallbilds; eine Vorverarbeitungseinheit (20), welche dafür vorgesehen ist, eine Anzahl von zweidimensionalen (2D)Bildern von dem 3D-Ultraschallbild zu erzeugen und die 2D-Bilder zu normalisieren, um normalisierte 2D-Bilder zu erzeugen; eine Randextraktionseinheit (30), welche in der Lage ist, Wavelet-transformierte Bilder der normalisierten 2D-Bilder in einer Vielzahl von Maßstäben zu bilden, wobei die Randextraktionseinheit des weiteren dafür vorgesehen ist, durch Berechnen des Durchschnitts der Wavelet-transformierten Bilder in einer Vielzahl von Maßstäben Randbilder zu bilden und die Randbilder zu begrenzen; eine Kontrollpunktermittlungseinheit (40), welche dafür vorgesehen ist, Kontrollpunkte unter Verwendung einer Support-Vektormaschine (SVM) basierend auf den normalisierten 2D-Bildern, den Wavelet-transformierten Bildern und den durch einen Schwellenwert begrenzten Randbildern zu ermitteln; und eine Rendereinheit (50), welche dafür vorgesehen ist, 3D-Volumendaten des Zielobjekts durch 3D-Rendern basierend auf den Kontrollpunkten zu erzeugen.

2. Ultraschallabbildungssystem nach Anspruch 1, wobei das Zielobjekt eine Prostata ist.

3. Ultraschallabbildungssystem nach Anspruch 1, wobei die Vorverarbeitungseinheit dafür vorgesehen ist, einen Durchschnitt und eine Standardabweichung der 2D-Bilder zu normalisieren, um die normalisierten 2D-Bilder zu erzeugen.

4. Ultraschallabbildungssystem nach Anspruch 3, wobei die Rendereinheit (50) dafür vorgesehen ist, wenigstens eines der normalisierten 2D-Bilder, der Wavelet-transformierten Bilder und der durch einen Schwellenwert begrenzten Randbilder basierend auf den Kontrollpunkten zu rendern.

5. Ultraschallabbildungssystem nach Anspruch 3, wobei die Kontrollpunktermittlungseinheit (40) dafür vorgesehen ist, die Kontrollpunkte wie folgt zu ermitteln:

   Anordnen einer Vielzahl von radialen Linien um einen Mittelpunkt des Zielobjekts in den durch einen Schwellenwert begrenzten Randbildern;
   Auswählen erster Kandidatenpunkte mit einer Helligkeit größer als Null auf jeder der radialen Linien;
   Festlegen von inneren und äußeren Fenstern um jeden der ersten Kandidatenpunkte;
   Vergleichen von Durchschnitten der Helligkeit in inneren und äußeren Fenstern in dem Wavelet-transformierten Bild bei einem vorbestimmten Maßstab;
   Auswählen zweiter Kandidatenpunkte mit einem größeren Helligkeitsdurchschnitt in dem äußeren Fenster als in dem inneren Fenster unter den ersten Kandidatenpunkten auf jeder der radialen Linien; Erzeugen von Merkmalsvektoren der zweiten Kandidatenpunkte in dem normalisierten 2D-Bild und Normalisieren der Komponenten der Merkmalsvektoren; Trainieren der SVM durch Verwenden der normalisierten Merkmalsvektoren;
   Auswählen von dritten Kandidatenpunkten mit der größten Helligkeit unter den zweiten Kandidatenpunkten auf jeder der radialen Linien in den durch einen Schwellenwert begrenzten Randbildern unter Verwendung der trainierten SVM;
   Nachregeln von Positionen der dritten Kandidatenpunkte basierend auf einer Basiskontur des Zielobjekts; und
   Ermitteln eines Randteils des Zielobjekts mit der größten Helligkeit innerhalb eines vorbestimmten Abstands unter den nachgeregelten Kandidatenpunkten als die Kontrollpunkte.

6. Verfahren zum Extrahieren von 3D-Volumendaten eines Zielobjekts, welches Folgendes aufweist:

   Bilden einer Anzahl von zweidimensionalen (2D) Bildern aus einem dreidimensionalen (3D) Bild; Normalisieren der 2D-Bilder, um normalisierte 2D-Bilder zu erzeugen;
   Erzeugen von Wavelet-transformierten Bildern der normalisierten 2D-Bilder in einer Vielzahl von Maßstäben;
   Bilden von Randbildern durch Berechnen des Durchschnitts der Wavelet-transformierten Bilder in einer Anzahl von Maßstäben;
   Begrenzen der Randbilder mit einem Schwellenwert; Ermitteln von Kontrollpunkten unter Verwendung einer Support-Vektormaschine (SVM) basierend auf den normalisierten 2D-Bildern, den Wavelet-transformierten Bildern und den mit einem Schwellenwert begrenzten Randbildern; und
   Erzeugen von 3D-Volumendaten des Zielobjekts durch 3D-Rendern basierend auf den Kontrollpunkten.

**EP 1 772 103 B1**

**7.** Verfahren nach Anspruch 6, wobei das Zielobjekt eine Prostata ist.

**8.** Verfahren nach Anspruch 6, wobei das Normalisieren der 2D-Bilder das Normalisieren eines Durchschnitts und einer Abweichung der Helligkeit in den 2D-Bildern beinhaltet.

**9.** Verfahren nach Anspruch 8, wobei die 3D-Volumendaten durch Rendern von wenigstens einem der normalisierten 2D-Bilder, der Wavelet-transformierten Bilder und der durch einen Schwellenwert begrenzten Randbilder basierend auf den Kontrollpunkten erzeugt werden.

**10.** Verfahren nach Anspruch 9, wobei das Ermitteln der Kontrollpunkte Folgendes beinhaltet:

Anordnen einer Vielzahl von radialen Linien um einen Mittelpunkt des Zielobjekts in dem durch einen Schwellenwert begrenzten Randbildern; Auswählen erster Kandidatenpunkte mit einer Helligkeit größer als Null auf jeder der radialen Linien;
Festlegen von inneren und äußeren Fenstern um jeden der ersten Kandidatenpunkte;
Vergleichen von Durchschnitten der Helligkeit in inneren und äußeren Fenstern in dem Wavelet-transformierten Bild bei einem vorbestimmten Maßstab;
Auswählen zweiter Kandidatenpunkte mit einem größeren Helligkeitsdurchschnitt in dem äußeren Fenster als in dem inneren Fenster unter den ersten Kandidatenpunkten auf jeder der radialen Linien; Erzeugen von Merkmalsvektoren der zweiten Kandidatenpunkte in dem normalisierten 2D-Bild und Normalisieren der Komponenten der Merkmalsvektoren; Trainieren der SVM durch Verwenden der normalisierten Merkmalsvektoren;
Auswählen von dritten Kandidatenpunkten mit der größten Helligkeit unter den zweiten Kandidatenpunkten auf jeder der radialen Linien in den durch einen Schwellenwert begrenzten Randbildern unter Verwendung der trainierten SVM;
Nachregeln von Positionen der dritten Kandidatenpunkte basierend auf einer Basiskontur des Zielobjekts; und Ermitteln eines Randteils des Zielobjekts mit der größten Helligkeit innerhalb eines vorbestimmten Abstands unter den nachgeregelten Kandidatenpunkten als die Kontrollpunkte.

**Revendications**

**1.** Système d'imagerie ultrasonore servant à former des données volumiques 3D d'un objet cible, comprenant :

une unité de création d'images tridimensionnelles (3D) (10) servant à créer une image ultrasonore 3D ;
une unité de prétraitement (20) adaptée pour former plusieurs images bidimensionnelles (2D) à partir de l'image ultrasonore 3D et pour normaliser les images 2D pour former des images 2D normalisés;
une unité d'extraction de contour (30) adaptée pour former des images transformées par ondelettes des images 2D normalisées à plusieurs échelles, l'unité d'extraction de contour étant en outre adaptée pour former des images de contour en prenant la moyenne des images transformées par ondelettes à plusieurs échelles et pour seuiller les images de contour ;
une unité de détermination de points de contrôle (40) adaptée pour déterminer des points de contrôle à l'aide d'une machine à vecteurs de support (SVM) sur la base des images 2D normalisées, des images transformées par ondelettes et des images de contour seuillées ; et
une unité de rendu (50) adaptée pour former des données volumiques 3D de l'objet cible par un rendu 3D sur la base des points de contrôle.

**2.** Système d'imagerie ultrasonore de la revendication 1, dans lequel l'objet cible est une prostate.

**3.** Système d'imagerie ultrasonore de la revendication 1, dans lequel l'unité de pré traitement est adaptée pour normaliser un écart moyen et un écart standard desdites images 2D pour former ies images 2D normalisées.

**4.** Système d'imagerie ultrasonore de la revendication 3, dans lequel l'unité de rendu (50) est adaptée pour faire le rendu d'au moins une des images 2D normalisées, des images transformées par ondelettes et des images de contour seuillées sur la base des points de contrôle.

**5.** Système d'imagerie ultrasonore de la revendication 3, dans lequel l'unité de détermination de points de contrôle (40) détermine les points de contrôle par:

la disposition de plusieurs lignes radiales autour d'un centre de l'objet cible dans les images de contour seuillées ;
la sélection de premiers points candidats possédant une brillance supérieure à zéro sur chacune des lignes radiales ;
la mise en place de fenêtres intérieures et extérieures autour de chacun des premiers points candidats ;
la comparaison des moyennes de brillance des fenêtres intérieures et extérieures dans l'image transformée par ondelettes à une échelle prédéterminée ;
la sélection de deuxièmes points candidats ayant une plus grande moyenne de brillance dans la fenêtre extérieure que dans la fenêtre intérieure parmi les premiers points candidats sur chacune des lignes radiales ;
la génération de vecteurs d'éléments des deuxièmes points candidats dans l'image 2D et la normalisation de composantes des vecteurs d'éléments ;
l'instruction de la SVM à l'aide des vecteurs d'éléments normalisés :

la sélection de troisièmes points candidats possédant la plus grande brillance parmi les deuxièmes points candidats sur chacune des lignes radiales dans les images de contour seuillées à l'aide de la SVM instruite;
le réajustement des positions des troisièmes points candidats sur la base d'un contour de base de l'objet cible ; et
la détermination d'une partie de contour de l'objet cible qui présente la plus grande brillance dans un rayon prédéterminé parmi les troisièmes points candidats réajustés en tant que points de contrôle.

**6.** Procédé pour extraire des données volumiques 3D d'un objet cible comprenant :

la formation de plusieurs images bidimensionnelles (2D) à partir d'une image tridimensionnelle (3D) ;
la normalisation des images 2D pour créer des images 2D normalisées ;
la formation d'images transformées par ondelettes des images 2D normalisées à plusieurs échelles ;
la formation d'images de contour en prenant la moyenne des images transformées par ondelettes à plusieurs échelles ;
le seuillage des images de contour ;
la détermination de points de contrôle à l'aide d'une machine à vecteurs de support (SVM) sur la base des images 2D normalisées, des images transformées par ondelettes et des images de contour seuillées ; et
la formation de données volumiques 3D de l'objet cible par un rendu 3D sur la base des points de contrôle.

**7.** Procédé de la revendication 6, dans lequel l'objet cible est une prostate.

**8.** Procédé de la revendication 6, dans lequel la normalisation des images 2D comprend la normalisation d'une moyenne de brillance et d'un écart de brillance dans les images 2D.

**9.** Procédé de la revendication 8, dans lequel la donnée volumique 3D est formée par un rendu d'au moins une des images 2D normalisées, les images transformées par ondelettes et les images de contour seuillées sur la base des points de contrôle.

**10.** Procédé de la revendication 9, dans lequel la détermination des points de contrôle comprend:

la disposition d'une pluralité de lignes radiales autour d'un centre de l'objet cible dans les images de contour seuillées ;
la sélection de premiers points candidats possédant une brillance supérieure à zéro sur chacune des lignes radiales ;
la mise en place de fenêtres intérieures et extérieures autour de chacun des premiers points candidats ;
la comparaison des moyennes de brillance dans des fenêtres intérieures et extérieures dans l'image transformée par ondelettes à une échelle prédéterminée ;
la sélection de deuxièmes points candidats possédant une plus grande moyenne de brillance dans la fenêtre extérieure que dans la fenêtre intérieure parmi les premiers points candidats sur chacune des lignes radiales ;
la génération de vecteurs d'éléments des deuxièmes points candidats dans les images 2D normalisées et la normalisation des composantes des vecteurs d'éléments ;
l'instruction de la SVM à l'aide des vecteurs d'éléments normalisés ;
la sélection de troisièmes points candidats possédant la plus grande brillance parmi les deuxièmes points candidats sur chacune des lignes radiales dans l'image de contour seuillée à l'aide de la SVM instruite ;
le réajustement des positions des troisièmes points candidats sur la base d'un contour de base de l'objet cible ; et
la détermination d'une partie de contour de l'objet cible possédant la plus grande brillance dans un rayon prédéterminée parmi les troisièmes points candidats réajustés en tant que points de contrôle.

## FIG. 1

100

10

20

30

| 3D IMAGE<br>PROVIDING<br>UNIT | → | PRE-PROCESSING<br>UNIT | → | EDGE<br>EXTRACTION<br>UNIT |

40

| CONTROL<br>POINT DETERMINING<br>UNIT | → | 3D RENDERING<br>UNIT | → | 3D PROSTATE<br>VOLUME DATA |

50

FIG. 2

FIG. 3A

PROSTATE

FIG. 3B

PROSTATE

# FIG. 4A

PROSTATE

FIG. 4B

PROSTATE

FIG. 5

# FIG. 6A

PROSTATE

FIG. 6B

PROSTATE

# FIG. 6C

PROSTATE

# FIG. 6D

PROSTATE

## FIG. 7A

PROSTATE

115H

FIG. 7B

0                                                 199

FIG. 8A

PROSTATE

FIG. 8B

FIG. 9

PROSTATE

FIG. 10

PROSTATE

## FIG. 11A

PROSTATE

## FIG. 11B

PROSTATE

## FIG. 11C

PROSTATE

## FIG. 11D

PROSTATE

FIG. 11E

PROSTATE

## FIG. 11F

PROSTATE

FIG. 12